## Europäisches Patentamt

## European Patent Office

## Office européen des brevets

(19)

(11) Veröffentlichungsnummer: **0 260 393**
**B1**

(12)

# EUROPÄISCHE PATENTSCHRIFT

(45) Veröffentlichungstag der Patentschrift:
**03.10.90**

(51) Int. Cl.⁵: **A61M 1/00, A61M 5/14**

(21) Anmeldenummer: **87109533.7**

(22) Anmeldetag: **02.07.87**

(54) Entlüftungsventil.

(30) Priorität: **18.09.86  DE 8625003 U**

(43) Veröffentlichungstag der Anmeldung:
**23.03.88 Patentblatt 88/12**

(45) Bekanntmachung des Hinweises auf die Patenterteilung:
**03.10.90 Patentblatt 90/40**

(84) Benannte Vertragsstaaten:
**AT BE CH DE ES FR GB IT LI NL SE**

(56) Entgegenhaltungen:
**EP-A- 0 093 272**
**FR-A- 2 321 906**

(73) Patentinhaber: **B. Braun Melsungen AG, Carl-Braun Strasse, D-3508 Melsungen(DE)**

(72) Erfinder: **Zahn, Jörn, Hermann-Mattern-Strasse 8, D-3500 Kassel(DE)**
Erfinder: **Schmidt, Klaus-Joachim, Brandenburger Strasse 16, D-3501 Ahnatal(DE)**

(74) Vertreter: **Selting, Günther, Dipl.-Ing. et al, Deichmannhaus am Hauptbahnhof, D-5000 Köln 1(DE)**

## Beschreibung

Die Erfindung bezieht sich auf ein Entlüftungsventil nach dem ersten teil vom Anspruch 1.

Entlüftungsventile dienen dazu, medizinische Geräte bei Blutfüllung zu entlüften, damit beispielsweise in Anwendung bei einer Punktionskanüle Blut in eine transparente Kammer des Kanülenansatzes strömen kann und den Punktionserfolg sichtbar macht. Solche Entlüftungsventile werden als "Blutfängerstopfen" bezeichnet und bestehen aus einem rohrförmigen Teil, dessen inneres Ende an die Leitung bzw. Kanüle angeschlossen ist, während sein äußeres Ende einen Verschluß aufweist, der gasdurchlässig ist, jedoch den Austritt von Blut verhindert. Bei einem bekannten Entlüftungsventil (DE-A 32 16 472) ist der gasdurchlässige Verschluß an zusammenwirkenden Flächen des rohrförmigen Teiles und einer Kappe durch mindestens eine schraubenförmige Nut realisiert. Beim Einströmen von Blut in das rohrförmige Teil entweicht durch die schraubenförmige Nut Luft nach außen und in der schraubenförmigen Nut koagulierendes Blut verhindert einen Blutaustritt aus dem Blutfängerstopfen, damit der Anwender sich nicht mit Blut infizieren kann. Günstig ist bei diesem Entlüftungsventil, daß die Gefahr des vorzeitigen Zusetzens der Entlüftungskanäle mit Blut gering ist, so daß der Blutrückfluß nicht gestaut wird und die Blutrückflußkontrolle gewährleistet ist. Ferner ist es aus der Praxis bekannt, Entlüftungsventile zur Verwendung als Blutsperre am Ende des rohrförmigen Teiles mit einem hydrophoben Filterstück auszustatten, das gasdurchlässig ist und dessen hydrophobe Beschaffenheit den Austritt von Blut aus dem Entlüftungsventil verhindert.

Bei den letztgenannten Entlüftungsventilen als Blutsperre ist das innere Ende des Kanals des rohrförmigen Teiles offen, so daß bei der Punktion eines Blutgefäßes das Blut mit Venendruck direkt gegen die Innenfläche des hydrophoben Filterstückes trifft. Dies ist nachteilig, weil das hydrophobe Material des Filterstückes von dem in seinen Poren koagulierenden Blut sofort abgedichtet wird, so daß keine Luft mehr entweichen kann und durch Stauung der Blutrückfluß unverzüglich unterbrochen wird. Der unterbrochene Blutrückfluß bei gestauten Venen hat zur Folge, daß die Blutrückflußkontrolle unwirksam ist und der Punkteur den Punktionserfolg nicht beurteilen kann, was zum Abbruch des Eingriffes zwingt.

Der Erfindung liegt die Aufgabe zugrunde, das Entlüftungsventil mit hydrophobem Filterstück so auszubilden, daß die Funktionsfähigkeit der Blutrückflußkontrolle zuverlässig erhalten bleibt, damit der Patient durch ggf. unnötige Punktionswiederholungen nicht strapaziert wird.

Diese Aufgabe wird erfindungsgemäß dadurch gelöst, daß das rohrförmige Teil im Bereich des inneren Endes des Kanals ein zentrales Prallelement aufweist.

Das zentrale Prallelement bewirkt, daß der unter Druck aus einer Kanüle oder einer medizinischen Leitung schießende Strahl nicht gegen das hydrophobe Filterstück, sondern gegen das Prallelement trifft und von diesem aus seiner geraden Bahn abgelenkt wird. Das hydrophobe Filterstück wird damit von Blut im wesentlichen freigehalten und behält seine Gasdurchlässigkeit, so daß der Blutrückfluß nicht zum Stillstand kommt und in der transparenten Kammer des Kanülenansatzes od. dgl. der Blutrückfluß den positiven Punktionserfolg zuverlässig anzeigt. Der Patient wird geschont, weil der Punkteur den Punktionserfolg stets richtig beurteilen kann und keine unnötigen Punktionen durchführt. Im übrigen verbessert das zentrale Prallelement nicht nur die Funktionsfähigkeit des Entlüftungsventils bei der Blutrückflußkontrolle, sondern es trägt auch zur Herabsetzung der Infektionsgefahr für den Anwender und den Patienten bei, weil der Kanal in dem rohrförmigen Teil sich infolge des zentralen Prallelementes nur sehr langsam mit Blut füllt und das hydrophobe Filterstück durch die verzögerte Blutbenetzung seine Schutzwirkung gegen Kontamination länger als bisher erfüllt.

In vorteilhafter Ausgestaltung der Erfindung ist das Prallelement als Prallplatte ausgebildet, die unter Belassung von Durchlässen zum Kanal an dem inneren Ende des rohrförmigen Teiles befestigt ist. Die Prallplatte kann kreisförmig sein und einen Durchmesser aufweisen, der kleiner als der lichte Kanalquerschnitt ist und es kann die Prallplatte mit radialen Armen versehen sein, die an der Stirnkante des rohrförmigen Teiles befestigt sind. Der aus der medizinischen Leitung oder Kanüle gegen die Prallplatte treffende Blutstrahl wird abgelenkt und gelangt erst nach Passieren der Durchlässe zwischen den radialen Armen in den Kanal des rohrförmigen Teils. Die Durchlässe zwischen den radialen Armen sind groß genug, um den freien Luftdurchtritt zum hydrophoben Filterstück solange freizugeben, bis die transparente Kammer des Kanülenansatzes oder dergleichen zur Blutrückflußkontrolle gefüllt ist. Alternativ kann die kreisförmige Prallplatte einen Durchmesser aufweisen, der etwa dem lichten Kanalquerschnitt entspricht und es können an der Prallplatte axiale Arme vorgesehen sein, die an der Stirnkante des rohrförmigen Teiles befestigt sind. Während im erstgenannten Falle die Durchlässe zwischen den Armen axial verlaufen, sind sie im zweiten Falle radial gerichtet, d.h. die Abschirmung des Kanals gegen den aus dem Kanülenende austretenden Blutstrahl wird verstärkt und die Möglichkeit des Zusetzens der Durchlässe mit koagulierendem Blut wird verringert.

Das rohrförmige Teil ist vorteilhafterweise als Außenkonus ausgebildet, der mit radialem Abstand von einem zylindrischen Mantel umgeben ist. Der Außenkonus wird in einen Innenkonus des Kanülenansatzes bzw. seiner transparenten Kammer oder ein entsprechendes Anschlußstück einer anderen medizinischen Leitung klemmend eingesteckt. Der zylindrische Mantel bildet einen Kontaminationsschutz beim Anfassen des Entlüftungsventils zu seiner Abnahme von der Leitung oder der Kanüle, wenn diese z. B. mit einem Flüssigkeitsübertragungssystem oder dergleichen verbunden werden soll.

In der Zeichnung sind Ausführungsbeispiele der Erfindung schematisch dargestellt. Es zeigen:

Fig. 1 einen Längsschnitt eines auf das Ende einer transparenten Kammer eines Kanülenansatzes aufgesteckten Entlüftungsventils,

Fig. 2 eine Draufsicht auf eine Ausführungsform des Prallelementes,

Fig. 3 einen Längsschnitt der Anordnung nach Fig.2 in vergrößertem Maßstab und

Fig. 4 eine weitere Ausführungsform eines Prallelementes.

Das Entlüftungsfilter 10 weist ein rohrförmiges Teil 11 mit konischer Außenfläche und einem durchgehenden zylindrischen Kanal 12 auf. Das rohrförmige Teil 11 ist mit radialem Abstand von einem zylindrischen Mantel 13 umgeben, der im wesentlichen gleiche Länge wie das rohrförmige Teil 11 hat und auf der Außenfläche mit mehreren längsgerichteten Rippen 14 versehen ist. Das äußere Ende des Kanals 12 mündet in eine konisch erweiterte Öffnung 15, die von einem axialen Kragen 16 umgeben ist. Zwischen der Innenwand des Kragens 16 und der Öffnung 15 befindet sich eine Schulter 17, auf der ein Filterstück 18 in Form eines sehr dünnen hydrophoben Plättchens fest haftet. Das Plättchen ist so dünn, daß der Kragen 16 um ein Vielfaches der Dicke des Plättchens über dieses vorsteht und einen Kontaminationsschutz gegen Fingerberührung bildet.

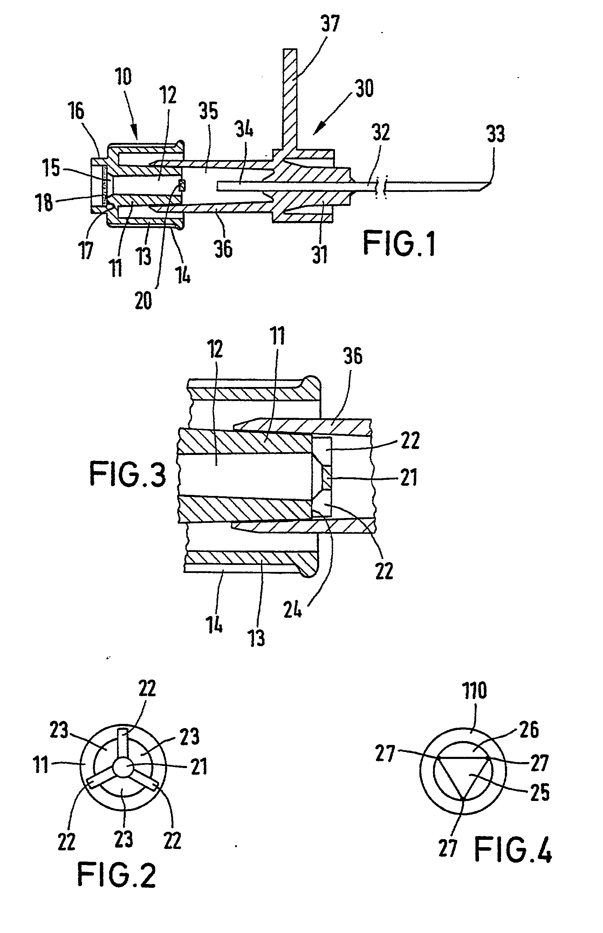

An dem inneren Ende des rohrförmigen Teiles 11 ist ein Prallelement 20 angebracht, das dem Kanal 12 zentral vorgesetzt ist und an seinem Umfang Durchlässe gegen den Kanal 12 freiläßt. Gemäß dem Beispiel nach Fig. 2 besteht das Prallelement 20 aus einer kreisförmigen Prall platte 21, deren Durchmesser kleiner ist als der lichte Querschnitt des Kanals 12 und von der symmetrisch drei radiale Arme 22 ausgehen. Zwischen den radialen Armen 22 verbleiben axiale Durchlässe 23. Das gesamte Entlüftungsventil kann im Spritzgußverfahren hergestellt sein und es können die radialen Arme 22, wie in Fig. 3 erkennbar, so profiliert sein, daß die dem Kanal 12 zugewandte Fläche der Prallplatte 21 der Stirnkante 24 des rohrförmigen Teiles ein kleines Stück vorgelagert ist, damit der Durchgang in Richtung des Kanals 12 zum Luftdurchlaß vergrößert wird.

Das Entlüftungsventil 10 ist gemäß Fig. 1 mit einem Kanülenansatz 30 verbunden. Dieser besteht im wesentlichen aus einem Trägerteil 31, in dem eine Stahlkanüle 32 mit angeschärfter Spitze 33 so befestigt ist, daß ihr patientenfernes stumpfes Ende 34 in eine koaxiale Kammer 35 mit transparenter Wandung 36 hineinragt. Die Wandung 36 der zylindrischen Kammer 35 ist an ihrem offenen Ende mit einem Innenkonus versehen, in den der Außenkonus 11 des Entlüftungsventils 10 klemmend einsteckbar ist. Dieser Zustand ist in Fig. 1 dargestellt. Eine Griffplatte 37, die von dem Trägerseil 31 quer zur Längsachse absteht, dient der Erleichterung der Handhabung bei der Punktion eines Blutgefäßes.

Wenn mit der Stahlkanüle 32 ein Blutgefäß punktiert wird, steigt mit Venendruck Blut in der Stahlkanüle 32 hoch und schießt aus der Öffnung des Kanülenendes 34 heraus, wobei es geradlinig auf die Prallplatte 21 auftrifft. Da diese zentral zu dem Kanal 12 angeordnet ist, lenkt sie den Blutstrahl zur Seite ab und verhindert, daß er unmittelbar zu dem Filterstück 18 gelangt und das hydrophobe Material abdichtet. Während die Kammer 35 sich mit Blut füllt, kann Luft durch die Durchlässe 23 im Umfangsbereich der Prallplatte 21 hindurchtreten und durch das unbeeinträchtigte trockene Filterstück 18 entweichen. Auf diese Weise ist gewährleistet, daß der Blutrückfluß bei gestauten Venen nicht unterbrochen wird und der Punkteur den Punktionserfolg zuverlässig beurteilen kann.

Gemäß Fig. 4 ist das Prallelement 20 als dreieckige Platte 25 gestaltet, die an dem inneren Ende eines rohrförmigen Teils 110 in die lichte Öffnung seines Kanals 26 eingesetzt und mit ihren drei Ecken 27 an dem rohrförmigen Teil 110 befestigt ist. Die zentrale Prallplatte 25 lenkt auch in diesem Falle den ersten Blutstrahl aus der Kanüle 32 ab, während die drei teilkreisförmigen Durchlässe an den Seiten der Prallplatte 25 bei der Blutfüllung verdrängte Luft gegen die Auslaßöffnung 15 durchlassen. Während die Prallplatte 21 des Beispiels der Fig. 2 und 3 der Stirnkante 24 des rohrförmigen Teiles 11 vorgesetzt ist, schließt die Prallplatte 25 im wesentlichen bündig mit der Stirnkante des rohrförmigen Teils 110 ab. Die eine oder die andere Version kann in Abhängigkeit von dem Abstand der Öffnung des Kanülenendes 34 zu dem Prallelement 20 und je nach Anwendungszweck günstig sein.

**Patentansprüche**

1. Entlüftungsventil als Blutsperre für medizinische Leitungen oder Kanülen, bei welchem der Kanal (12) eines rohrförmigen Teiles (11) an seinem der Leitung bzw. der Kanüle zugewandten inneren Ende der mit der Leitung bzw. Kanüle (32) verbunden ist, und an seinem der Leitung bzw. der Kanüle abgewandten äußeren Ende von einem hydrophoben Filterstück (18) verschlossen ist, **dadurch gekennziechnet,** daß das rohrförmige Teil (11) im Bereich des der Leitung bzw. der Kanüle zugewandten inneren Endes des Kanals (12) ein zentrales Prallelement (20) aufweist.

2. Entlüftungsventil nach Anspruch 1, **dadurch gekennzeichnet,** daß das Prallelement (20) als Prallplatte (21) ausgebildet ist, die unter Belassung von Durchlässen (23) zum Kanal (12) an dem inneren Ende des rohrförmigen Teiles (11) befestigt ist.

3. Entlüftungsventil nach Anspruch 2, **dadurch gekennzeichnet,** daß die Prallplatte (21) kreisförmig ist und einen Durchmesser aufweist, der kleiner als der lichte Kanalquerschnitt ist und daß die Prallplatte (21) mit radialen Armen (22) versehen ist, die an der Stirnkante (24) des rohrförmigen Teiles (11) befestigt sind.

4. Entlüftungsventil nach Anspruch 2, **dadurch gekennzeichnet,** daß die Prallplatte kreisförmig ist und einen Durchmesser aufweist, der etwa dem lichten Kanalquerschnitt entspricht und daß die Prallplatte mit axialen Armen versehen ist, die an der Stirnkante (24) des rohrförmigen Teiles (12) befestigt sind.

5. Entlüftungsventil nach Anspruch 3 oder 4, **dadurch gekennzeichnet,** daß drei Arme (22) symmetrisch um den Umfang der Prallplatte (21) verteilt sind.

6. Entlüftungsventil nach einem der Ansprüche 1 bis 5, **dadurch gekennzeichnet**, daß das rohrförmige Teil (11) als Außenkonus ausgebildet ist, der mit radialem Abstand von einem zylindrischen Mantel (13) umgeben ist.

## Claims

1. A ventilating valve for use as a blood barrier for medical conduits or cannulas, wherein the channel (12) of a tubular member (11) has its inner end, facing the conduit or the cannula, connected to the conduit or the cannula (32), and wherein said channel is closed at its outer end, facing away from the conduit or the cannula, by a hydrophobic filter member (18), characterized in that said tubular member (11) has a central battle element (20) provided in the area of the inner end of said channel (12) facing the conduit or the cannula.

2. The ventilating valve of claim 1, characterized in that said baffle element (20) is formed as a baffle plate (21) connected to the inner end of said tubular member (11), while leaving passages (23) to the channel (12).

3. The ventilating valve of claim 2, characterized in that said baffle plate (21) is circular in shape and has a diameter that is smaller than the free channel cross section and that said baffle plate (21) has radial arms (22) fastened to the front edge (24) of said tubular member (11).

4. The ventilating valve of claim 2, characterized in that said baffle plate is circular in shape and has a diameter that is smaller than the free channel cross section and that said baffle plate has axial arms (22) fastened to the front edge (24) of said tubular member (11).

5. The ventilating valve of claim 3 or 4, characterized in that three arms (22) are distributed symmetrically about the circumference of the baffle plate (21).

6. The ventilating valve of one of claims 1 to 5, characterized in that said tubular member (11) is formed as an outer cone that is surrounded by a radially spaced cylindrical jacket (13).

## Revendications

1. Soupape d'évacuation d'air formant système empêchant l'écoulement du sang pour des conduits ou des canules médicales et dans laquelle le canal (12) d'une partie tubulaire (11) est relié, par son extrémité intérieure tournée vers le conduit ou la canule (32), à ce conduit ou cette canule et est fermé, à son extrémité extérieure tournée à l'opposé du conduit ou de la canule, par un élément filtrant hydrophobe (18), caractérisée en ce que l'élément tubulaire (11) possède un élément central à impact (20) situé dans la zone de l'extrémité intérieure du canal (12), tournée vers le conduit ou la canule.

2. Soupape d'évacuation d'air selon la revendication 1, caractérisée en ce que l'élément à impact (20) est réalisé sous la forme d'une plaque à impact (21), qui est fixée à l'extrémité intérieure de l'élément tubulaire (11), tout en laissant subsister des passages (23) en direction du canal (12).

3. Soupape d'évacuation d'air selon la revendication 2, caractérisé en ce que la plaque à impact (21) est de forme circulaire et un diamètre inférieur à la section transversale libre du canal et que la plaque à impact (21) est pourvue de bras radiaux (22), qui sont fixés sur le bord frontal (24) de l'élément tubulaire (11).

4. Soupape d'évacuation d'air selon la revendication 2, caractérisé en ce que la plaque à impact est de forme circulaire et possède un diamètre qui correspond approximativement à la section transversale libre du canal et que la plaque à impact est pourvue de bras axiaux qui sont fixés sur le bord frontal (24) de l'élément tubulaire (12).

5. Soupape d'évacuation d'air selon la revendication 3 ou 4, caractérisée en ce que trois bras (22) sont répartis symétriquement sur le pourtour de la plaque à impact (21).

6. Soupape d'évacuation d'air selon l'une des revendications 1 à 5, caractérisée en ce que la partie tubulaire (11) est réalisée sous la forme d'un cône extérieur, qui est entouré, à une certaine distance radiale, par une enveloppe cylindrique (13).

FIG.1

FIG.3

FIG.2

FIG.4